# EUROPÄISCHE PATENTSCHRIFT

(11) **EP 1 534 734 B1**
(45) Veröffentlichungstag und Bekanntmachung des Hinweises auf die Patenterteilung: **07.05.2008**
(21) Anmeldenummer: 03794978.1
(22) Anmeldetag: 01.09.2003
(51) Int. Cl.: C07K 1/00

(54) **VERFAHREN ZUR SYNTHESE UND SELEKTIVEN BIOKATALYTISCHEN MODIFIZIERUNG VON PEPTIDEN, PEPTIDMIMETIKA UND PROTEINEN**
METHOD FOR THE SYNTHESIS AND SELECTIVE BIOCATALYTIC MODIFICATION OF PEPTIDES, PEPTIDE MIMETICS AND PROTEINS
PROCEDE DE SYNTHESE ET DE MODIFICATION BIOCATALYTIQUE SELECTIVE DE PEPTIDES, DE SUBSTANCES MIMETIQUES DE PEPTIDES ET DE PROTEINES

(30) Priorität: 30.08.2002 DE 10240098
(43) Veröffentlichungstag der Anmeldung: 01.06.2005
(73) Patentinhaber: Roche Diagnostics GmbH, 68305 Mannheim (DE); F. Hoffmann-La Roche AG, 4070 Basel (CH)
(72) Erfinder: BORDUSA, Frank, 06242 Rossbach (DE); WEHOFSKY, Nicole, 03417 Leipzig (DE); RUDOLPH, Rainer, 06120 Halle/Saale (DE)
(74) Vertreter: Isenbruck, Günter
(86) Internationale Anmeldenummer: PCT/EP2003/009694
(87) Internationale Veröffentlichungsnummer: WO 2004/024751

(56) Entgegenhaltungen:
- EP-A- 1 201 657
- EP-A- 1 348 767
- WO-A-02/26701
- WO-A-02/26772
- SUMMERS CATHERINE A ET AL: "Protein renaturation by the liquid organic salt ethylammonium nitrate" PROTEIN SCIENCE, Bd. 9, Nr. 10, Oktober 2000 (2000-10), Seiten 2001-2008, XP009022169 ISSN: 0961-8368
- ARMSTRONG D W ET AL: "Ionic liquids as matrixes for matrix-assisted laser desorption/ionization mass spectrometry." ANALYTICAL CHEMISTRY. UNITED STATES 1 AUG 2001, Bd. 73, Nr. 15, 1. August 2001 (2001-08-01), Seiten 3679-3686, XP001156233 ISSN: 0003-2700
- KULLMANN W: "PROTEASES AS CATALYSTS FOR ENZYMIC SYNTHESES OF OPIOID PEPTIDES" JOURNAL OF BIOLOGICAL CHEMISTRY, Bd. 255, Nr. 17, 1980, Seiten 8234-8238, XP002263435 ISSN: 0021-9258
- PARK S ET AL: "Improved preparation and use of room-temperature ionic liquids in lipase-catalyzed enantio- and regioselective acylations." THE JOURNAL OF ORGANIC CHEMISTRY. UNITED STATES 14 DEC 2001, Bd. 66, Nr. 25, 14. Dezember 2001 (2001-12-14), Seiten 8395-8401, XP002263436 ISSN: 0022-3263
- VAN RANTWIJK F ET AL: "Biocatalytic transformations in ionic liquids" TRENDS IN BIOTECHNOLOGY, ELSEVIER PUBLICATIONS, CAMBRIDGE, GB, Bd. 21, Nr. 3, März 2003 (2003-03), Seiten 131-138, XP004412613 ISSN: 0167-7799
- PARK S. ET AL: "Biocatalysis in ionic liquids - Advantages beyond green technology." CURRENT OPINION IN BIOTECHNOLOGY, (2003) 14/4 (432-437). , XP002263437

## Beschreibung

Die vorliegende Erfindung betrifft ein Verfahren zur enzymatischen Synthese und/oder selektiven Modifizierung von Peptiden, Peptidmimetika und/oder Proteinen unter Verwendung ionischer Flüssigkeiten sowie die Verwendung von ionischen Flüssigkeiten als ausschließliches Reaktionsmedium oder in Kombination mit Wasser und/oder organischen Lösungsmitteln zur Unterdrückung hydrolytischer und proteolytischer Nebenreaktionen.

Die Synthese und selektive Modifizierung von Peptiden, Peptidmimetika und Proteinen besitzt zunehmende Bedeutung für das systematische Studium von Struktur-Funktionsbeziehungen der Polypeptide als funktionelle Genprodukte und trägt entscheidend zur Entdeckung neuer wirksamer Therapeutika bei (vgl. H.-D. Jakubke, Peptide: Chemie und Biologie, Spektrum Akademischer Verlag, Heidelberg, Berlin, Oxford, 1996). Wesentliche Probleme ihrer Synthese oder selektiven Modifizierung sind jedoch die fehlende Selektivität und Universalität chemischer Verfahren und die Substratlimitation bzw. das Auftreten zahlreicher Nebenreaktionen bei der Verwendung von Enzymen als Katalysatoren.

Grundsätzlich lassen sich für die Synthese von Peptiden, Peptidmimetika und Proteinen die in der Peptidchemie entwickelten chemischen Methoden einsetzen. Diese unterliegen jedoch erheblichen Limitationen mit steigender Komplexität der Produkte. Während Peptide mit einer durchschnittlichen Kettenlänge von 50-60 Aminosäuren direkt durch Festphasenpeptidsynthese zugänglich sind, führt eine weitere Kettenverlängerung wegen der nicht in jedem Fall quantitativen Kupplungsausbeuten häufig zur Akkumulation einer Vielzahl von Nebenprodukten, die sowohl zu einer Verringerung der Syntheseausbeuten fuhren wie auch die Reinigung des gewünschten Produktes erschweren bzw. verhindern. Gegenwärtige Methoden zur Synthese längerer Polypeptide bzw. von Proteinen beruhen daher auf der Kondensation von synthetisch bereitgestellten Peptidfragmenten, wobei jedoch die Verknüpfung vollgeschützter Peptidfragmente wegen der häufig sehr geringen Löslichkeit der Edukte nur in Ausnahmefällen möglich ist

Die basierend auf dem erstmals 1953 vorgeschlagenen Konzept der Molekülklammer für chemische CN-Ligationen von ungeschützten Peptidfragmenten (T. Wieland et aL, Annalen 1953, 583, 129; M. Brenner et al., Helv. Chim. Acta 1957, 40,1497) entwickelten Methoden des Amin- und Thioleinfangs (D.S. Kemp et al., J. Org Chem. 1975, 40, 3465; N. Fotouhi et al., J. Org. Chem. 1989, 54, 2803), der natürlichen chemischen Ligation (M. Schnölzer, S.B.H. Kent, Science 1992, 256,221; P.E. Dawson et al., Science 1994, 266, 776) oder auch der Aldehyd-Methode (C.-F. Liu, J.P. Tam, Proc. Natl. Acad Sci. USA 1994, 91, 6584) verlaufen zwar selektiv, erfordern aber für deren Realisierung ganz bestimmte N- bzw. C-terminale Aminosäurereste, so dass ihre Anwendbarkeit sequenzspezifischen Voraussetzungen unterliegt Bei der z.Z. favorisierten nativen chemischen Ligation erfolgt die Verknüpfung eines synthetischen Peptids mit einer C-terminalen Thioestergruppierung mit einem zweiten Peptid oder Protein, das einen N-terminalen Cysteinrest enthalten muß. Unter Nutzung der Erkenntnisse des Proteinspleißens wurde die native chemische Ligation zu einer Intein-vermittelten Protein-Ligation (*expressed protein ligation,* EPL; vgL u. a. T.W. Muir et al., Proc. Natl. Acad Sci. USA 1998, 95, 6705; G.J. Cotton et al., J. Am. Chem. Soc. 1999, 1.21, 1100), bei der die Thioestergruppierung der Carboxykomponente aus einem rekombinanten Protein, das mit einem spaltungskompetenten Intein fusioniert ist und durch thiolytische Spaltung gebildet wird, weiterentwickelt Neben der Notwendigkeit eines Cysteinrestes am N-Temzinus der Aminokomponente liegt ein weiterer genereller Nachteil in der nicht anszuschließendea partiellen Epimerisierung des C-terminalen Aminosäurerestes, da der sich bildende Thioester (zumindest bei Einsatz von Thiophenol als Katalysator) nicht nur erst nach der Umesterung, sondern auch direkt von der terminalen α-Aminogruppe der zugesetzten Aminokomponente nukleophil angegriffen werden kann.

Katalytische Syntheseverfahren bieten den Vorteil einer höheren Flexibilität hinsichtlich der zu synthetisierenden Peptidbindung, obgleich aus der Natur zumindest bislang keine universelle Peptidligase mit präparativer Relevanz bekannt ist. So zeigen katalytische Antikörper (vgl. u.a. P.G. Schultz, RA Lerner, Science 1995, 269,1835; G. MacBeath, D. Hilvert, Chem. Biol. 1996, 3, 433; D.B. Smithrub et al., J. Am Chem. Soc. 1997, 119, 278) CN-Ligaseaktivität, ebenso wie synthetische Peptidligasen basierend auf einem coiled-coil-Motif von GCN4 (K. Severin et al., Nature 1997, 389, 706) bzw. auf einer Peptidmatrize bestehend aus einem stark sauren coiled-coil-Peptid (S. Yao, J. Chmielewski, Biopolymers 1999, 51, 370). Bei allen diesen Fällen handelt es sich zweifelsohne um interessante Ansatzpunkte für das Design von Peptidligasen, die für Ligationen allerdings spezielle Voraussetzungen erfordern und deren allgemeine Anwendbarkeit folglich sehr stark limitiert ist. Es ist bekannt, dass Proteasen als Katalysatoren für die enzymatische Synthese von Peptiden eingesetzt werden können, beispielsweise die Proteasen Papain und Chymotrypsin für die Synthese von Ovoid-Peptiden (W. Kullmann, Journal of Biological Chemistry 1980 Bd. 255, Nr. 17, 8234-8238).Die Nutzung des reversen Katalysepotentials von Peptidasen (vgl. u.a W. Kullmann, Enzymatic Peptide Synthesis, CRC Press, Boca Raten, 1987; H.-D Jakubke, Enzymatic Peptide Synthesis, in: The Peptides: Analysis, Synthesis, Biology, Vol. 9, (Eds.: S. Udenfriend, J. Meienhofer), Academic Press, New York, 1987, Chapter 3) bietet zwar die prinzipielle Möglichkeit, Peptidsegmente unter speziellen Voraussetzungen enzymatisch zu verknüpfen, doch ist weder die Irreversibilität der geknüpften speziellen Peptidbindung garantiert noch sind unerwünschte proteolytische Spaltungen in den zu verknüpfenden Segmenten bzw. im Endprodukt a priori auszuschließen, wenn sich dort potentielle Spaltstellen für die eingesetzte Peptidase befinden. Obgleich durch Reengineering verschiedener Peptidasen, wie z.B. Subtilisin das Katalysepotential zur Peptidbindungsknüpfung verbessert und auch durch anspruchsvolle Fragmentkondensationen (vgl. u.a D.Y. Jackson et al., Science 1994, 266, 243) belegt werden konnte, können dadurch die oben geschilderten Nachteile nicht ausgeräumt werden. Das für CN-Ligationen von Peptid- und Proteinsegmenten entwickelte Substratmimetika-Konzept (F. Bordusa et al., Angew. Chem.1997, 109, 2583; Review: F. Bordusa, Braz. J. Med. Biol. Res. 2000, 72, 469) hat zwar den Vorteil der Irreversibilität, bedarf aber ebenfalls der Verwendung synthetischer, proteolytisch inaktiver Proteasevarianten, um kompetitive Spaltungen innerhalb der zur verknüpfenden Biopolymere zu verhindern.

WO 02/26772 offenbart Verfahren zur katalytischen selektiven Modifikation von Peptiden und Proteinen unter Verwendung von u.a. Trypsin und Chymotrypsin.

Zur Modifizierung von Peptiden, Peptidmimetika und Proteinen spielten - und spielen noch immer - chemische Verfahren (vgL T. Imoto, H. Yamada, Chemical Modification, in Protein Function. A Practical Approach (T.B. Creighton, ed.) pp. 247-277, IltL Press, 1989; G.B. Means, RE. Feeney, Chemical Modification of Proteins, Holden-Day, 1971) eine bedeutende Rolle in der Proteinforschung. So ist trotz des schnellen Fortschritts der NMR-Technik, die im letzten Jahrzehnt eine vollständige Signalzuordnung und somit eine Aufklätung der 3D-Strukturen von Proteinen bis zu 150 - 200 Aminosäurebausteinen (ohne Modifizierung) ermöglichte, die chemische Modifizierung weiterhin auch ein Werkzeug zur Raumstrukturbestimmung in Lösung, da große Proteine der NMR-Strukturanalyse nicht zugänglich sind und die Röntgenstrukturanalyse Proteinkristalle erfordert, die in sehr vielen Fällen nicht erhalten werden können.

Da N-terminale α-Aminogruppen bevorzugte Ziele von selektiven Modifizierungen sind, erlauben die ε-Aminogruppen ubiquitär in Proteinen und Peptiden vorkommender Lysinreste keine gezielte Einführung von Marker- und Reportergruppen an den N-Terminus. Chemische Acylierungsreaktionen werden mit Anhydriden oder vorrangig mit aktiven Estern, wie z.B. *N-*Hydroxysuccinimid- oder 4-Nitrophenylestem durchgeführt, womit aber auch andere Seitenkettenfunktionen proteinogener Aminosäurereste reagieren können und damit eine selektive lr-Modifihtion ausschließen. Lediglich der Phenylacetyl-Rest wurde spezifitätsdeterminiert durch die Penicillin-Acylase in Umkehrung der nativen Wirkung als Schutzgruppe für Aminosäuren im Rahmen von Peptidsynthesen enzymatisch eingeführt (R. Didziapetris et al., FEBS Lett. 1991, 287, 31) und durch das gleiche Enzym wieder abgespalten (vgl. Review: A. Reidel, H. Waldmann, J. prakt. Chem. 1993, 335, 109). Abgesehen von dieser direkten Schutzgruppeneinfiihrung wurden nur solche Methoden beschrieben, die auf einer .. Übertragung bereits N-taminal markierter Aminosäure- oder Peptidderivate mit Peptidasespezifischen Aminosäureresten in der P₁-Position unter der Katalyse von Peptidasen beruhen und zwangsläufig keine Irreversibilität aufweisen. Eine Ausnahme hiervon ist das ursprünglich für CN-Ligationen von Peptid- und Proteinsegmenten entwickelte Substratmimetika-Konzept (F. Bordusa et al., Angew. Chem 1997, 109, 2583; Review: F. Bordusa, Braz. J. Med Biol. Res. 2000, 72, 469). Diese Methodik hat zwar den Vorteil der direkten und selektiven Einführung von Marker- und Reportergruppen, bedarf aber, wie bereits erwähnt, der Verwendung synthetischer, proteolytisch inaktiver Proteasevarianten als Biokatalysatoren, um kompetitive Spaltungen der zu markierenden Biopolymere zu verhindern.

Die Unterdrückung hydrolytischer und proteolytischer Nebenreaktionen von zur Synthese und Modifizierung von Peptiden, Peptidmimetika und Proteinen eingesetzten Hydrolasen kann neben einem gezielten Enzymengineering auch durch Manipulationen am Reaktionsmedium erfolgen. In der Literatur beschrieben ist die Verwendung von einphasigen Mischungen aus Wasser und organischen Lösungsmitteln, aus analogen biphasischen Systemen im Falle einer Nichtmischbarkeit von Wasser und organischem Lösungsmittel, von reinen organischen Lösungsmitteln mit praktisch keinem oder nur sehr geringem Wasseranteil, von gefrorenen oder unterkühlten wässrigen oder organischen Systemen, von superkrischen Lösungen und von heterogenen eutektischen Mischungen mit praktisch keinem oder nur sehr geringem Lösungsmittelanteil (vgl. u.a. W. Kullmann, Enzymatic Peptide Synthesis, CRC Press, Boca Raton, 1987; H.-D. Jakubke, Enzymatic Peptide Synthesis, in: The Peptides: Analysis, Synthesis, Biology, Vol. 9, (Eds.: S. Udenfriend, J. Meienhofer), Academic Press, New York, 1987, Chapter 3). Allerdings bewirken praktisch alle diese Methoden eine oft dramatische Verringerung der Enzymaktivität- bzw. Stabilität und bedürfen teilweise eines erheblichen apparativen Aufwandes. Hinzu kommt, dass nur wenige überhaupt hinsichtlich ihrer Verwendbarkeit für die Synthese und Modifizierung längerkettiger Biopolymere untersucht worden sind Aber auch in solchen Fällen konnte keines dieser Verfahren bislang die für eine routinemässige Anwendung geforderte Effizienz und Universalität nachweisen.

Eine neue Klasse von Lösungsmitteln repräsentieren Salze, die einen niedrigen Schmelzpunkt aufweisen. Für diese; auch als ionische Flüssigkeiten bezeichneten Lösungsmittelsysteme, konnte in initialen Studien ein stabilisierender Einfluß auf Proteine und Enzyme nachgewiesen werden (Review: C.M. Gordon, Appl. Catal. A: Gen. 2001, 222, 101). Es wurde gezeigt, dass die Verwendung der ionischen Flüssigkeit Ethylammonium-Nitrat die Rückfaltung eines reduzierten/denaturierten Hühner-Eiweiss-Proteins (Lysozym) begünstigt und der Aggregation des denaturierten Proteins entgegenwirken kann (C.A. Summers, R.A. Flowers, Protein Science 2000, Band 9, Nr. 10, 2001-2008). Zudem ist bekannt, dass ionische Flüssigkeiten gute Lösungsmittel-Eigenschaften für Peptide und Proteine aufweisen und daher zur Beschichtung von Matrizen für UV-MALDI-Analysen eingesetzt werden können (D. W. Armstrong et al., Analytical Chemistry, United States 2001, Band 73, Nr. 15, 3679-3686). Auch ist bekannt, dass Lipasen durch die Einwirkung von ionischen Flüssigkeiten nicht inaktiviert werden. Bestimmte ionische Flüssigkeiten zeigen Polaritäten, die polaren organischen Flüssigkeiten ähnlich sind. Es wurde gezeigt, dass die Lipase-katalysierte Acetylierung von 1-Phenylethanol ebenso schnell in ionischen Flüssigkeiten wie in Toluen war (S. Park, Journal of Organic Chemistry 2001, Band 66, Nr. 25, 8395-8401). An einfachen Modellreaktionen mit Lipasen und Galactosidasen wurde zudem gezeigt, dass diese Flüssigkeiten einen positiven Effekt auf die Reaktionsgeschwindigkeit und teilweise auch auf die Selektivität der enzymatischen Reaktionen besitzen (U. Kragl et al., Chimica Oggi 2001, 19, 22; T.L. Husum et al., Biocatal. Biotrans. 2001, 19, 331; S.H. Schofer et al., Chem. Commun. 2001,425). Am Beispiel eines einfachen Aminosäureester-Substrdtes konnte weiterhin demonstriert werden, dass auch die Serinproteasen Chymotrypsin und Subtilisin in Reaktionssystemen mit hohem Anteil an solchen Flüssigkeiten enzymatisch aktiv ist und sowohl die Hydrolyse des Esters wie auch dessen Transesterifizierung katalysiert (JA Laszlo, D.L. Compton, Biotechnol. Bioeng. 2001, 75, 181; T.L. Husum et al., Biocatal. Biotrans. 2001, 19, 331). Anhand der Synthese von 2-Asp-Phe-0Me aus Z-Asp-OH und H-Phe-OMe durch die Metalloprotease Thermolysin konnte zudem die prinzipielle Eignung von ionischen Flüssigkeiten für die Protease-katalysierte Verknüpfung zweier Aminosäuren unter gleichgewichtskontrollierten Synthesebedingungen demonstriert werden (M. Erbeldinger et al., Biotechnol. Prog. 2000, 16, 1131). Bislang völlig unbekannt hingegen ist, ob in solchen Flüssigkeiten Peptidfragmente selektiv durch Proteasen in einer kinetisch-kontrollieren Reaktionsfnhrung verknüpft werden können und ob unter diesen Bedingungen eine selektive Einführung von Reporter- und Markergruppierungen an den N-Terminus von Peptiden und Proteinen durch Proteasen katalysiert wird In gleicher Weise ist unklar, welchen Einfluß ionische Flüssigkeiten auf das Ausmaß proteolytischer Nebenreaktionen an den Edukten sowie hydrolytischer Nebenreaktionen am eingesetzten Estersubstrat besitzen.

Der vorliegenden Erfindung liegt die Aufgabe zugrunde, ein Verfahren zur enzymatischen Synthese und Modifizierung von Peptiden, Peptidmimetika und Proteinen bereitzustellen, welches die Nachteile der im Stand der Technik beschriebenen Verfahren überwindet Weiterhin ist es Aufgabe der vorliegenden Erfindung, ein Verfahren bereitzustellen, bei dem eine sequenzunabhängige Synthese, insbesondere Ligation und N-terminale Modifizierung, ohne proteolytische und hydrolytische Nebenreaktionen an den Edukten bzw. den Reaktionsprodukten regio- und stereoselektiv erfolgt.

Die Aufgabe wird erfindungsgemäß durch ein Verfahren zur Synthese von Peptiden, Peptidmimetika und/oder Proteinen und/oder zur selektiven N-terminalen Modifizierung von Peptiden, Peptidmimetika und/oder Proteinen gelöst, mit den Schritten:
a) Bereitstellen einer Aminokomponente, wobei die Aminokomponente mindestens eine Aminosäure aufweist,
b) Bereitstellen einer Carboxykomponente, wobei die Carboxykomponente eine Abgangsgruppe an der Carboxylgruppe aufweist, und die Carboxykomponente eine Verbindung mit mindestens einer Aminosäure ist oder eine Verbindung mit mindestens einer Marker- oder Reportergruppe,
c) Umsetzen der Aminokomponente und der Carboxykomponente in einem Reaktionsmedium, welches ein oder mehrere ionische Flüssigkeiten aufweist, in Gegenwart einer Protease, Peptidase und/oder Hydrolase, wobei zwischen der Aminokomponente und der Carboxykomponente unter Abspaltung der Abgangsgruppe eine Peptidbindung gebildet wird.

In einer bevorzugten Ausführungsform ist vorgesehen, dass das erfindungsgemäße Verfahren weiter den Schritt umfasst:
d) Isolieren oder Anreichern des erhaltenen Peptids, Peptidmimetikums und/oder Proteins nach an sich bekannten Verfahren.

Weiterhin betrifft die vorliegende Erfindung die Verwendung von ionischen Flüssigkeiten als ausschließliches Lösungsmittel oder in Kombination mit Wasser und/oder organischen Lösungsmitteln zur Synthese und/oder N-teiminalen Modifzierung von Peptiden, Peptidmimetika und/oder Proteinen. Die vorliegende Erfindung betrifft auch die Verwendung einer Protease, Peptidase und/oder Hydrolase zur Synthese und/oder N-terminalen Modifzierung von Peptiden, Peptidmimetika und Proteinen, wobei das Peptid, Peptidmimetikum und Protein oder deren N-terminal markierte Spezies durch Ligation einer Aminokomponente und einer Carboxykomponente hergestellt wird und die Carboxykomponente eine Abgangsgruppe aufweist

Weitere Ausführungsformen ergeben sich aus den Unteransprüchen und der nachfolgenden Beschreibung.

Erfindungsgemäß sind unter Peptiden Kondensationsprodukte von Aminosäuren mit etwa 2-10 Aminosäuren zu verstehen. Unter Polypeptiden sind erfindungsgemäß Kondensationsprodukte von Aminosäuren mit etwa 10 -100 Aminosäuren zu verstehen und der Begriff Proteine wird erfindungsgemäß für Kondensationsprodukte von Aminosäuren verwendet, die mehr als etwa 100 Aminosäuren aufweisen, wobei der Übergang zwischen den beiden Begriffen in der Literatur als fließend angesehen wird

Mit Peptidmimetika sind erfindungsgemäß Verbindungen bezeichnet, die die biologische Aktivität eines Peptides nachahmen oder antagonisieren ohne selbst einen klassischen Peptidaufbau ausschließlich aus kodierten Aminosäuren zu besitzen. Beispiele für Peptidmimetika sind neben gänzlich nichtpeptidischen organischen Verbindungen (z.B. das aus cyclo-aliphatischen und aromatischen Strukturen aufgebaute Morphin bzw. Naloxon) auch solche, die modifizierte Aminosäuren (z.B. N-, α- und β-alkylierte Aminosäuren; C_{α}-C_{β} und N-C_{β} cyclisierte Aminosäuren; Peptide mit modifizierten Seitenketten wie z.B. α-,β-dehydrogenierte Aminosäuren, Nitrotyrosin etc.) aufweisen, sowie cyclische Peptidanaloga (Cyclisierung von N-Terminus mit C-Terminus bzw. Aminosäureseitenkette; Cyclisierung von C-Terminus mit Aminosäureseitenkette bzw. Cyclisierung von Aminosäureseitenketten mit Aminosäureseitenkette) und Peptide mit modifizierten Peptidbindungen wie z.B. Thioamiden, Ketomethylenen, Ethylenen, Methylenaminen oder auch Retro-Inverso-Derivaten u.ä. Retro-Iaverso-Derivate sind Verbindungen mit der Peptidrückgrat-Struktur R-C-NH-CO-C-R`, bei denen die Stellung der Aminofunktion und der Carbonsäurefunktion im Vergleich zu normalen Peptidbindungen vertauscht ist, während normale Peptidbindungen die Struktur R-C-CO-NH-C-R' aufweisen.

Ionische Flüssigkeiten sind, im Gegensatz zu Salzschmelzen, bei niedrigen Temperaturen (<100°C) schmelzende Salze, die ausschließlich aus Ionen bestehen (Lit S. z.B. T. Welton, Chem. Rev. 1999, 2071-2083). Nach dieser Definition ist Wasser also keine ionische Flüssigkeit Charakteristische Merkmale sind ihre niedrige Symmetrie, geringe intermolekulare Wechselwirkungen und eine gute Ladungsverteilung. Typische Kationen enthalten quaternisierte Heteroatome wie etwa quaternisierte Ammonium- oder quaternisierte Phosphonium-Ionen. Untergruppen sind z.B. N-alkylierte Imidazolium-Ionen wie 1-Ethyl-3-methylimidazolium, 1-Butyl-3-Methylimidazoliurm N-alkylierte Pyridiniumionen wie 4-Methyl-N-butyl-pyridinium bzw. analog substituierte Ammonium- und Phosphoniumionen. Typische Anionen können sowohl anorganischer als organischer Natur sein wie z.B. Chlorid, Bromid, Cloralurninat, Nitrat, Benzosulfonat, Triflat, Tosylat oder auch Tetrafluorborat.

Ausgehend von dem bisher im Stand der Technik beobachteten Phänomen, dass der vollständige aber auch teilweise Ersatz von Wasser als Reaktionsmedium durch reaktionsinerte Lösungsmittel zu einem Aktivitätsverlust bis hin zur Inaktivierung des Enzyms führt, lag der vorliegenden Erfindung die überraschende Erkenntnis zugrunde, dass es möglich ist, eine mit einer Abgangsgruppe versehenen Carboarykomponente, wobei die Carboxykomponente ein Peptid, Peptidmimetika; Protein bzw. eine Marker- oder Reportergruppierung darstellt, mit hoher Syntheserate und Selektivität enzymkatalysiert, unter Verwendung von ionischen Flüssigkeiten als ausschließliches Lösungsmittel oder in Kombination mit Wasser und/oder organischen Lösungsmitteln als Reaktionsmedium, mit einer Aminokompomponente, die vorzugsweise ein Peptid, Peptidmimetika und Protein darstellt, zu verknüpfen.

Dabei war weiterhin überraschend, dass die typischerweise ablaufenden Nebenreaktionen wie die Hydrolyse der Bindung zwischen Carboxykomponente und Abgangsgruppe sowie die Proteolyse von der Spezifität des Enzyms entsprechenden Peptidbindungen in den Edukten bzw. den Produkten der Reaktion praktisch nicht ablaufen. Die Carboxykomponente wird mit der Abgangsgruppe typischerweise dadurch versehen, dass die Abgangsgruppe ester-, thioester- oder amidartig mit der C-tenninalen Carboxylfunktion der Carboxykomponente verknüpft wird. Dem erfindungsgemäßen Verfahren liegt somit die überraschende Erkenntnis zugrunde, dass ionische Flüssigkeiten oder Mischungen daraus im Gegensatz zu praktisch allen anderen organischen Lösungsmitteln einen die Syntheseaktivität des Enzyms begünstigenden Einfluss aufweisen und gleichzeitig die typischerweise durch das Lösungsmittel Wasser vermittelten unerwünschten Nebenreaktionen praktisch vollständig unterdrücken. Durch den kombinierten Einsatz ionischer Lösungsmittel mit Carboxykomponenten, die enzymspezifische Abgangsgruppen enthalten, kann weiterhin eine Unabhängigkeit der Syntheseaktivität von der ursprünglichen Substratspezifität des Enzyms erreicht werden, was die synthetische Anwendungsbreite des Verfahrens entscheidend erhöht.

Es wurde gefunden, dass alle bislang untersuchten Serin- und Cysteinproteasen das vorstehend beschriebene Verhalten zeigen und somit im Rahmen der erfindungsgemäßen Verfahren zur Synthese und Modifizierung von Peptiden, Peptidmimetika und Proteinen besonders geeignet sind. Weitere geeignete Enzyme können im Rahmen von Screening-Prozessen unter Verwendung geeigneter Modellreaktionen, wie sie auf der Grundlage der hierin offenbarten technischen Lehre durchgeführt werden können, erhalten werden.

Im Rahmen eines derartigen Screening-Prozesses wird dabei so vorgegangen, dass die Syntheseaktivität von Proteasen, Peptidasen und/oder Hydrolasen in ionischen Flüssigkeiten oder Mischungen daraus mittels synthetischer ModeUreaktionen untersucht wird Hierzu wird im einfachsten Fall eine aus einer Aminosäure bestehende Carboxykomponente (herkömmliche Carboxykomponenten oder Substratmimetikum) mit einer Aminokomponente, im einfachsten Fall ein Amid einer Aminosäure, bevorzugter Weise aber einem Peptid, und der zu testenden Protease, Peptidase oder Hydrolase inkubiert. Die Toleranz von ionischen Flüssigkeiten durch das Enzym wird durch Produktbildung im Verlauf der nachfolgenden Inkubationsphase angezeigt. Die Produktbildung selbst kann beispielsweise mittels HPLC oder anderer chromatographischer Trennmethoden analysiert werden.

Erfindungsgemäß sind als Proteasen Cysteinproteasen oder Serinproteasen bevorzugt Es können aber grundsätzlich alle anderen bekannten Arten von Proteasen, also auch Aspartatproteasen oder Metalloproteasen eingesetzt werden. Als weitere Hydrolasegruppen kommen insbesondere Lipasen oder Esterasen in Frage. Als Peptidasen (EC 3.4.11-3.4.19) können erfindungsgemäß ebenfalls die bekannten Peptidaseuntergruppen grundsätzlich eingesetzt werden. Zur Definition von Hydrolasen, Peptidasen und Proteinasen sei insbesondere auf Römpp Chemielexikon, 9. Auflage, 1989 -1992 verwiesen.

Ein weiterer Vorteil des erfindungsgemäßen Verfahrens gründet sich auf der Regiospezifität der verwendeten Enzyme und des nichtvorhandenen Racemisierungsrisikos gegenüber den meisten chemischen Verfahren. Dies ist insoweit vorteilhaft, als dass Edukte mit chiralen Zentren und anderen acylierbaren Funktionen ohne experimentell aufwendige und zu zusätzlichen Nebenreaktionen führenden temporären und selektiven Blockierungsmaßnahmen eingesetzt werden können. Ausnahmen sind lediglich die in einigen Fällen notwendige Einführung N-terminaler Schutzgruppen in die Carboxykomponente, die insbesondere dann erforderlich wird, wenn die N-terminale Sequenz der Carboxykomponente eine höhere Spezifität zum Enzym aufweist als die N-terminale Sequenz der Aminokomponente. Darüber hinaus existieren im Gegensatz zu den selektiven chemischen Methoden praktisch keine Beschränkungen hinsichtlich der Sequenz der in Reaktion tretenden Edukte,

Erfindungsgemäß können Proteasen, Peptidasen und/oder Hydrolasen als Enzyme eingesetzt werden. Diese weisen bevorzugt eine Selektivität oder Spezifität für die Abgangsgruppe und/oder bestimmte Aminosäuren oder Aminosäurebereiche der Carboxykomponente auf. Die Abgangsgruppe und/oder diese Aminosäuren oder Aminosäurebereiche können erfindungsgemäß bevorzugt von dem verwendeten Enzym natürlicherweise erkannte Verbindungen sein. Es kann sich erfindungsgemäß bevorzugt hierbei aber auch jeweils um strukturell ähnliche Verbindungen handeln (Substratmimetika).

Die Begriffe Aminokomponente und Carboxykomponente, wie sie hierin verwendet werden, werden relativ zu dem zu synthetisierenden Polypeptid definiert Dabei bezeichnet der Begriff der Aminokomponente eine chemische Verbindung, die mindestens eine Aminogruppe zur Verfügung stellt, die mit einer Carboxygruppe oder einem Derivat davon, bspw. einer Carboxygruppe, die mit einer Abgangsgruppe derivatisiert ist, unter Ausbildung einer Peptidbindung reagiert Bei der Aminokomponente handelt es sich bevorzugt um eine Aminosäure, bevorzugter um ein Polypeptid oder Protein. Im letzteren Falle weist das Polypeptid oder Protein sowohl ein Amino-Ende als auch ein Carboxy-Ende auf. Das Carboxy-Ende liegt dabei entweder ungeschützt oder geschützt vor. Zur Vermeidung einer Reaktion mit einer anderen reaktiven Gruppe, wie beispielsweise der Aminogruppe eines weiteren Moleküls der Aminokomponente, liegt die Carboxylgruppe der Aminokomponente typischerweise und bevorzugt in nicht-aktivierter Form vor. Es ist weiterhin bevorzugt, dass die *N*^{α}-Ammofunktion der Aminokomponente ungeschützt vorliegt, wobei diese *N*^{α}-Aminofunktion mit der Carboxyfunktion der Carboxykomponente reagiert.

Bei der Carboxykomponente handelt es sich bevorzugt um eine mit einer Carboxylfunktion versehenen Marker- oder Reportergruppe oder um eine Aminosäure oder, bevorzugter um ein Polypeptid oder Protein. Dabei ist die Carboxylfunktion oder -gruppe der Carboxykomponente, die mit der Aminogruppe, in der Regel der N-terminalen Aminogruppe, der Aminokomponente unter Ausbildung einer Peptidbindung reagiert, typischerweise und bevorzugt aktiviert.

Es ist erfindungsgemäß bevorzugt, dass die Abgangsgruppe der Carboxykomponente ausgewählt ist aus der Gruppe, umfassend unsubstituierte und substituierte -O-Alkyl-, -O-Aryl-, -S-Alkyl-, -S-Aryl-Reste, NH-Alkyl-, -NH-Aryl-, -N,N-Dialkyl-. -N,N-Diaryl- und -N-Aryl-N-Alkyl-Reste, wobei ebenfalls bevorzugt die Abgangsgruppe der Carboxykomponente durch ein oder mehrere Carbonsäurereste, Sulfonsäurereste oder Sulfonate substituiert ist. Der Begriff Alkyl umfasst in dieser Aufzählung auch Cycloalkyl und Heterocycloalkyl. Als Heteroatome kommen insbesondere N, O und S in Frage. Bevorzugt sind Cycloalkane oder Heterocycloalkane mit 5-6 Ring-Kohlenstoffatomen. Unter den Alkylresten sind n-Alkylreste und verzweigte Alkylreste erfindungsgemäß umfasst, bevorzugt sind hierunter n-Alkylreste mit 1- 5 C-Atomen

Der Begriff Aryl umfasst in dieser Aufzählung insbesondere substituiertes und unsubstituiertes Phenyl, das bevorzugt durch Guanidino und/oder Amidino am Phenylring substituiert ist. Der Begriff Aryl umfasst hier aber auch anellierte Ringsysteme, bevorzugt Biphenyl, und nicht anellierte Ringsystem wie Naphthyl, die wiederum bevorzugt duch Guanidine- und/oder Amidino-Gruppen substituiert sein können, und heteroanaloge Systeme wie Chinolin oder Isochonolin. Der Begriff Aryl umfasst hier auch heteroaromatische Verbindungen mit 5 - 6 Ringatomen, wobei ein oder mehrere Ringkohle,nstoffatome bevorzugt durch N, O und/oder S ersetzt sind. Beispiele sind Pyridino-, Thiophen- Furan-, Pyrazol- oder Imidazolreste.

Besonders bevorzugt ist die Abgangsgruppe der Carboxykomponente ein 4-Guanidinophenyl-, 4-Amidinophenyl-, 4-Guanidinophenylthio-oder 4-Amidinophenylthio-Rest oder eine hierzu strukturhomologe Verbindung.

Bei der Carboxykomponente bildet die Abgangsgruppe mit der Carboxygruppe der Carboxykomponente bevorzugt einen Ester oder ein Amid, bevorzugterweise an der C-terminalen Carboxylgruppe der Carboxykomponente. Wie bereits ausgeführt, wird durch die spezifische Erkennung der Abgangsgruppe letztendlich die enzymatische Aktivität des Enzyms dergestalt abgeändert, dass auch Peptidfragmente oder Marker- und Reportergruppen anstelle von Edukten mit enzymspezifischen Aminosäureresten durch die Protease, Hydrolase oder Peptidase verknüpft werden.

Für Argini.n-spezifische Proteasen, wie z.B. Trypsin, wurde für die 4-Guanidinophenylester-Abgangsgruppe eine derartige spezifitätsvennittelnde Wirkung festgestellt. Eine analoge Funktion ist auch für Amidinophenylester zu beobachten. Darüber hinaus besitzen aufgrund der Strukturhomologie die 4-Guanidinophenylthioester- und 4-Amidinophenylthioester-AnaIoga eine ähnliche Wirkung und weisen zudem Vorteile bei der chemischen Synthese auf. Strukturhomologe dieser Verbindungen kommen ebenfalls als spezifitätsvermittelnde Abgangsgruppen in Betracht

Als strukturhomologe Verbindungen gelten Derivate dieser Verbindungen mit einer basischen Gruppierung, wie beispielsweise Amino-, Amidino-, Guanidino- und Iminogruppierungen, die mit spezifitätsbestimmenden Aminosäureresten der Protease wechselwirken, d.h. insbesondere solchen Aminosäureresten, die direkt oder indirekt mit dem Substrat in Wechselwirkung treten bzw. solchen, die die katalytische Reaktion beeinflussen, und die einen aliphatischen oder aromatischen Grundkörper aufweisen mit z.B. einer Kettenlänge zwischen einer und sechs Methyleneinheiten oder Benzol-, Naphthalin- oder Indol-Grundkörpern zwischen der spezifischen basischen Gruppierung und der Ester- bzw. Amidfunktion als verknüpfendes Element zwischen der Carboxylgruppe der Carboxykomponente und der Abgangsgruppe. Im übertragenen Sinne gilt dies ebenfalls für Enzyme mit einer primären Spezifität für Glutaminsäure bzw. Asparaginsäure, wie z.B. die V8 Protease, mit dem Unterschied, dass statt der basischen Gruppierungen an den aliphatischen oder aromatischen Grundkörpern saure Gruppierung wie Carboxyl- und Sulfonsäuregruppen gebunden sind. In analoger Weise stellen Ester oder Amide, die lediglich aus den genannten Grundkörpern bestehen, also keine basischen oder sauren Gruppen aufweisen, Carboxylkomponenten für Enzyme mit einer bevorzugten Spezifität für hydrophobe Aminosäurereste, wie z.B. Chymotrypsin und Subtilisin, dar.

Bevorzugt wird erfindungsgemäß die Abgangsgruppe an die Spezifität der verwendeten Protease, Peptidase und/oder Hydrolase angepasst

In einer weiteren bevorzugten Ausführungsform weist die die Abgangsgruppe enthaltende Carboxykomponente die folgende Struktur auf:

Y-(Xaa)ₙ-R

wobei Y = eine N-terminale Schutzgruppe oder H ist,
Xaa = eine beliebige α-Aminosäure, β-Aminosäure oder ein Derivat derselben oder eine Marker- oder Reportergruppe ist,

R eine Abgangsgruppe ist, insbesondere eine Abgangsgruppe ist, die ausgewählt ist aus der Gruppe, umfasssend unsubstituierte und substituierte -O-Alkyl-, -O-Aryl-, -S-Alkyl-, - S-Aryl-Reste, bevorzugt 4-Guanidinophenyl-, 4-Amidinophenyl-, 4-Guanidinophenylthio-, 4-Amidinophenylthio-Reste, die jeweils durch Sulfonsäuregruppen oder Sulfonate substituiert sein können, sowie Strukturhomologe hiervon, n eine ganze Zahl von 1 bis 1000, bevorzugt 30 bis 500, noch bevorzugter 30 bis 250 ist

Hierbei sind die Begriffe Alkyl und Aryl wie oben definiert und umfassen wie oben auch Cycloalkane, Heterocycloalkane, anellierte Ringsystem und nicht anellierte Ringsysteme sowie die oben erwähnten bevorzugten Ausführungsformen.

Es ist ebenfalls bevorzugt, dass die Carboxykomponente eine Marker- oder Reportergruppe ist, ausgewählt aus Carboxygruppen enthaltenden Fluoreszenzmarkern wie Fluorescein, Rhodamin, Tetramethylrhodamin, 2-Aminobenzoesäure, Carboxygruppen enthaltenden Isotopenlabeln wie ¹³C-. ¹⁵N- und ¹⁷O-enthaltenden Aminosäuren oder Peptidfragmenten; Spinlabeln, wie Nitroxidlabel enthaltende Aminosäure und Fettsäurederivate; Biotin; Carboxygruppen enthaltenden vernetzenden Mittel (cross-linking agents) wie Diazoacetat, Diazopyruvat, p-Nitrophenyl-3-diazopynivat; 2-(1,2-Dithiolan-3-yl)acetat; N,N'-1,2-Phenylendimaleimid; N,N'-1,4-Phenylendimaleimid. Alle genannten Derivate besitzen eine Carboxygruppe, die vor der enzymatischen Reaktion mit einer der zuvor definierten Abgangsgruppen versehen sind. Insofern handelt es sich bei den genannten Fluoreszenzmarkern nicht um die kompletten Carboxykomponenten, sondern nur um den Teil, der auf die Aminokomponente übertragen wird

Durch die Verknüpfung der Aminokomponente und der Carboxykomponente wird ein Polypeptid oder selektiv modifiziertes Polypeptid oder Analogon desselben gebildet, wobei das C-terminale Ende der Aminokomponente dem C-terminalen Ende des Polypeptids entspricht und das Amino-terminale Ende der Carboxykomponente demAmino-Ende des unter dem Einfluss des Enzyms ligierten Polypeptids bzw. der eingeführten Marker- und Reportergruppe entspricht Die Länge des synthetisierten bzw. modifizierten Polypeptids beträgt mindestens zwei Aminosäuren. Typischerweise wird die Länge des erfindungsgemäß hergestellten Polypeptids oder Proteins eine Größe von 1 bis 1000 Aminosäuren, bevorzugter 30 bis 1000, noch bevorzugter 50 bis 600 und am bevorzugtesten 100 bis 300 Aminosäuren aufweisen.

Die Größe der Aminokomponente kann sowenig wie eine Aminosäure betragen. Eine obere Grenze der Länge der Aminokomponente ist nicht notwendigerweise gegeben, wird jedoch letzten Endes, wenn überhaupt, durch die Spezifität des verwendeten Enzyms sowie reaktionskinetischen Betrachtungen, wie beispielsweise Diffusionsgeschwindigkeit der Aminokomponente, bestimmt werden. Typische Größen der Aminokomponente sind dabei 1 bis 1000 Aminosäuren, bevorzugter 30 bis 500 Aminosäuren und bevorzugter 30 bis 250 Aminosäuren. Es ist jedoch auch im Rahmen der vorliegenden Erfindung, dass die Länge der Aminokomponente deutlich größer ist, insbesondere bei solchen Ausführungsformen des erfindungsgemäßen Verfahrens, bei denen eine sequenzielle Enzym- bzw. Protease-katalysierte Peptidfragmentügation erfolgt oder ein Protein als Edukt dient Die Länge beträgt dann bevorzugter Weise ein Vielfaches der vorstehend genannten Längenbereiche. Dabei ist es im Rahmen der vorliegenden Erfindung, dass die Aminokomponente größer, gleich oder kleiner als die Carboxykomponente ausgebildet ist, wobei als Kriterium hierzu in der Regel die Anzahl der die Aminokomponente bzw. die Carboxykomponente ausbildenden Aminosäuren herangezogen wird.

Es ist bevorzugt, dass die Carboxykomponente eine Größe von 1 bis 1000 Aminosäuren, bevorzugt 30 bis 500, noch bevorzugter 30 bis 250 Aminosäuren aufweist.

Bei dem erfindungsgemäßen Verfahren ist bevorzugter Weise vorgesehen, dass als Aminokomponente N-terminal ungeschützte Peptide, Peptidmimetika und Proteine eingesetzt werden.

Die Reaktion erfolgt bevorzugt in reinen ionischen Flüssigkeiten, wie z.B. 4-Methyl-N butyl-pyridinium-tetrafluorborat, mit keinem oder nur sehr geringem Wassergehalt (typischer Weise kleiner als 5%).

In einer weiteren Ausführungsform der Erfindung beträgt der Anteil der ionischen Flüssigkeiten im Reaktionsmedium 50 -100 Vol.-%, bevorzugt 70 -100 oder 80 -100 Vol.-%, bevorzugter 90- 100 Vol.-%, ebenfalls bevorzugt 95 bis 100 Vol.-%, 95 - 99 Vol.-% oder 97-99 Vol.-%.

Gemische aus ionischen Flüssigkeiten und organischen Lösungsmitteln mit und ohne Wasseranteil und weiteren Zusätzen, wie z.B. anorganischen Salzen, stellen ebenfalls Reaktionsmedien im Sinne der Erfindung dar, wobei es unerheblich ist, ob es sich um eine Lösung oder Suspension handelt. Als Zusätze können insbesondere eingesetzt werden: anorganische Salze, Pufferkomponenten, Reduktions- und Oxidationsmittel, Enzymaktivatoren, -modulatoren und inhibitoren, Tenside, Lipide, Polymere zur kovalenten bzw. adhesiven Immobilisierung von Proteinen (z.B. Polyethylenglycol, Methoxypolyethylenglycol oder Carboxymethylcellulose) und proteindenalurierende Agenzien wie SDS (Sodiumdodecylsulfat), Harnstoff oder Guanidinhydrochlorid.

Ein entscheidender Vorteil der Verwendung von Lösungsmittelgemischen kann in der Erhöhung oder Verringerung der Löslichkeit von Edukten oder Enzym bzw. in der Beeinflussbarkeit der Enymaktivität und --spezifität durch Anzahl, Art und Anteil der zusätzlich zur ionischen Flüssigkeit verwendeten Lösungsmittel liegen.

Erfindungsgemäß sind bevorzugt mit Wasser mischbare organische Lösungsmittel einsetzbar, wenn diese sich auch mit den ionischen Flüssigkeiten mischen. Andererseits sind erfindungsgemäß auch hydrophobe organische Lösungsmittel (z.B. Hexan oder Oktan) umfasst, die nicht mit Wasser mischbar sind, wobei das Lösungsmittelgemisch als biphasiges System vorliegt. Erfindungsgemäß ist auch die Verwendung von modifizierten inonischen Flüssigkeiten mit hydrophoben Alkylgruppen die sich entweder teilweise oder vollständig mit hydrophoben organischen Lösungsmitteln mischen, umfasst

Es ist erfindungsgemäß bevorzugt, dass ionische Flüssigkeiten eingesetzt werden, bei denen die Kationen Alkyl-Imidazolium-Ionen, Alkyl-Ammonium-Ionen, Alkyl-Pyridinium-Ionen und/oder Alkyl-Phosphoniuin-Ionen sind, wobei die Alkylierung jeweils vollständig ist, d.h. keines der genannten Heteroatome an ein Wasserstoff gebunden ist, die also quaternisiert sind.

Ebenfalls bevorzugt ist, dass die Alkylreste der ionischen Flüssigkeiten verzweigt oder unverzweigt sind und 1-20 C-Atome, bevorzugt 1-20 C-Atome, noch bevorzugter 4-6 C-Atome aufweisen. Besonders bevorzugt ist, wenn mindestens ein Alkylrest Methyl, Ethyl Propyl oder Butyl ist, insbesondere Butyl.

Als Anionen der ionischen Flüssigkeiten sind erfindungs gemäß Chlorid, Bromid, Chloraluminat, Nitrat, Benzolsulfonat, Triflat (Tiifluoimethansulfonat), Tosylat und/oder Tetrafluorborat bevorzugt einsetztbar.

Es ist erfindungsgemäß besonders bevorzugt, dass als ionische Flüssigkeiten 1-Ethyl-3-methylimidazolium-, 1 -Butyl-3-rnethylimidazolium- und/oder 4-Methyl-N-butyl-pyridinium-Salze eingesetzt werden, wobei das jeweilige Tetrafluorborat besonders bevorzugt ist.

Die Synthese der Carboxykomponenten mit der bevorzugter Weise Enzym-spezifischen Abgangsgruppe kann chemisch durch Kondensation des Acylrestes der Carboxykomponente mit der jeweiligen Abgangsgruppe (oder geeigneten Vorstufen) oder aber am polymeren Träger, z.B. durch Verwendung von Sulfamylbutyrylaminomethyl-Safety-Catch-Harzen (vgl. R.

Ingenito et al., J. Am Chem. Soc. 1999, 121, 11369) oder Oxim-Harzen (vgl. V. Cerovsky, F. Bordusa, J. Peptide Res. 2000,35,325; V. Cerovsky et al., ChemBioChem 2000, 2,126) mit synchroner Ester- bzw. Amid-Generierung und Peptidabspaltung erfolgen. Als ablösendes Nukleophil kommt die entsprechende alkoholische, phenolische, Mercapto- oder Aminogruppen-enthaltende Abgangsgruppe selbst, wie auch bereits vorgefertigte *N*^{α}-ungeschützte Aminosäureester bzw. -amide oder geeignete Vorstufen zum Einsatz. Alternativ kann die Synthese der Carboxykomponente auf gentechnischem Wege wie z.B. durch Verwendung der Intein-vermittelten Polypeptidestersynthese erfolgen (M. W. Southworth et aL, Biotechniques 1999, 27, 110). Die Anpassung der Abgangsgruppe kann durch die Wahl des Nukleophiles bei der Inteinspaltung oder aber nach bereits erfolgter Estergenerierung durch Umesterung in Lösung erfolgen.

Die Synthese der als Aminokomponenten eingesetzen Peptidfragmente ist in Lösung oder durch Verwendung konventioneller Fmoc- oder Boc-Syntheseprotokolle am polymeren Träger routinemäßig möglich. Üblicherweise ist die Synthese am polymeren Träger wegen der Vorteile bei der Aufreinigung der einzelnen Zwischenprodukte einer aufwendigeren Lösungssynthese vorzuziehen. Alternativ kann die Aminokomponente aus biologischem Material gewonnen oder durch gentechnische Verfahren mit anschließender Isolierung exprimiert werden.

Die erhaltenen synthetisierten oder markierten Produkte können mit üblichen Methoden der Peptid- und Proteinchemie separiert und gereinigt werden. Ggf. vorhandene Schutzgruppen können nach im Stand der Technik bekannten Verfahren entfernt werden.

Gemäß einer bevorzugten Ausf-uhrungsfoim der Erfindung kann eine gemäß den Schritten a) bis c) und ggf. d) hergestellte Verbindung als Aminokomponente und eine andere gemäß den Schritten a) bis c) und ggf. d) hergestellte Verbindung als Carboxykomponente eingesetzt werden, um größere Polypeptide oder Proteine aus definierten Bestandteilen aufzubauen, die je nach Verfahrensführung auch Marker- oder Reportergruppen aufweisen können.

Im Gegensatz zu anderen potentiell verwendbaren biokatalytischen Verfahren wird durch die erfindungsgemäße Verwendung von ionischen Flüssigkeiten oder Mischungen derselben in Kombination mit dem Einsatz von Enzymen und insbesondere Proteasen und Peptidasen eine hohe Syntheserate, Flexibilität, Syntheseeffizienz und Einfachheit in der Handhabung erreicht.

Die vorliegende Erfindung wird anhand der Zeichnungen und Beispiele veranschaulicht, aus denen sich weitere Merkmale, Ausführungsformen und Vorteile der Erfindung ergeben.

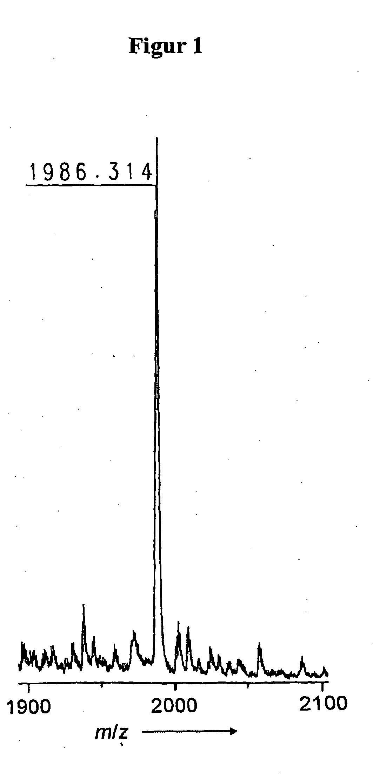

Dabei zeigt Fig. 1 ein ausgesuchtes MALDI-ToF Massenspektrum des unter Anwendung des erfindungsgemäßen Verfahrens und wie in Beispiel 4 beschrieben hergestellten biotinylierten Peptids.

### Beispiele

### Beispiel 1 - Einfluss des Anteiles der ionischen Flüssigkeit 4-Methyl-N-butyl-pyridinium tetrafluorborat auf die Trypsin-katalysierte Synthese von Di- und Tripeptiden (Bz, Benzoyl; OGp, 4-Guanidinophenylester).

1 ml Reaktionslösung, die die in Tab. 1 angegebenen Gemische aus 4-Methyl-N-butyl-pyridinium tetrafluorborat enthält sowie 0,1 M Hepes-Puffer pH 8,0, enthaltend 0,1 M NaCl und 0,01 M CaCl₂, 1,5% (v/v) 4-Methylmorpholin, 2 mM Bz-Phe-OGp, 20 mM Aminokomponente und 10 µM Trypsin enthält, wird bei 25 °C gerührt. Nach 30-120 min wird die Reaktionslösung mit 1 proz. Trifluoressigsäure in Methanol/Wasser (1:1, v/v) auf pH 2 gebracht Die Ausbeuten an Di- und Tripeptid wurden HPLC-analytisch bestimmt und sind in der nachfolgenden Tabelle 1 aufgeführt.

Die Synthese von Bz-Phe-OGp erfolgte analog des Syntheseprotokolles von M. Thormann et al., Biochemistry 1999, 38, 6056. Die verwendeten Aminokomponenten sind kommerziell erhältliche Produkte und sind in Tab 1 angegeben. Trypsin wurde von Fluka (Schweiz) bezogen.

**Tabelle 1:**

| Anteil an ionischer Flüssigkeit (v/v) | H-Leu-NH₂ | H-Gly-NH₂ | H-Met-NH₂ | H-Ser-NH₂ | H-Ala-Met-OH |
|---|---|---|---|---|---|
| 0 | 75,2 | 45,2 | 68,0 | 52,1 | 51,5 |
| 20 | 77,4 | 49,1 | 69,3 | 54,5 | 53,9 |
| 40 | 79,7 | 54,1 | 72,9 | 58,4 | 57,6 |
| 60 | 85,8 | 62,4 | 77,0 | 68,9 | 68,5 |
| 80 | 87,3 | 64,7 | 79,0 | 70,4 | 71,4 |

### Beispiel 2 - Einfluss der Art und des Anteiles zusätzlicher organischer Lösungsmittel auf die Trypsin-katalysierte Synthese von Bz-Phe-Leu-NH₂ ausgehend von Bz-Phe-OGp und H-Leu NH₂ in der ionischen Flüssigkeit 4-Methyl-N-butyl-pyridinium tetrafluorborat (Bz, Benzoyl; OGp, 4-Guanidinophenylester; MeOH, Methanol; DMSO, Dimethylsulfoxid; DMF, Dimethylformamid).

1 ml Reaktionslösung, die 4-Methyl-N butyl-pyridinium tetrafluorborat, 5% Wasser und die angegebenen Anteile an zusätzlichem organischen Lösungsmittel, 1,5% (v/v) 4-Methylmorpholin, 2 mM Bz-Phe-OGp, 20 mM Aminokomponente und 20, 40, 80, 200 µM Trypsin (mit steigendem Anteil an zusätzlichem organischen Lösungsmittel) enthält, wird bei 25 °C gerührt. Nach 30-120 min wird die Reaktionslösung mit 1 proz. Trifluoressigsäure in Methanol/Wasser (1:1, v/v) auf pH 2 gebracht Die Ausbeuten wurden HPLC-analytisch bestimmt und sind in der nachfolgenden Tabelle 2 aufgeführt.

**Tabelle 2:**

| Organisches Lösungsmittel | Produktausbeute (%) / Verhältnis ionische Flüssigkeit: organisches Lösungsmittel | | | |
|---|---|---|---|---|
| | 50 : 50 (v/v) | 60 : 40 (v/v) | 70 : 30 (v/v) | 80 : 20 (v/v) |
| MeOH | 93.2 | 93.7 | 93.8 | 94.7 |
| DMSO | 91.9 | 92.4 | 92.8 | 94.8 |
| DMF | 91.7 | 92.0 | 92.7 | 93.7 |

### Beispiel 3 - Trypsin-katalysierte Synthese von Polypeptiden mit enzym-spezifischen Spaltstellen ausgehend von Bz-Phe-OGp und Polypeptiden verschiedener Länge und Sequenz in der ionischen Flüssigkeit 4-Methyl-N-butyl-pyridinium tetrafluorborat.

Die individuellen enzym-spezifischen Aminosäurereste sind jeweils durch Fettdruck hervorgehoben (Bz, Benzoyl; OGp, 4-Guanidinophenylester).

1 ml Reaktionslösung, die 4-Methyl-N butyl-pyridinium tetrafluorborat, 5% Wasser, 1,5% (v/v) 4-Methylmorpholin, 2 mM Bz-Phe-OGp, 5 mM Aminokomponente und 10 µM Trypsin enthält, wird bei 25 °C gerührt. Aus Löslichkeitsgründen wurden die Reaktionen mit Methanol als zusätzliches organisches Lösungsmittel durchgeführt, wobei sowohl ein Anteil von 20% als auch 50% (v/v) Methanol verwendet wurde. Nach 30-120 min wird die Reaktionslösung mit 1 proz. Trifluoressigsäure in Methanol/Wasser (1:1, v/v) auf pH 2 gebracht Die mittels MALDI-ToF nach ihrer Isolierung aus der Reaktionslösung identifizierten Polypeptidproäukte sind mit den jeweils berechneten bzw. gefundenen Molekülmassen in Tabelle 3 aufgeführt.

**Tabelle 3:**

| Syntheseprodukt | berechnete Masse [g/mol]* | Gefundene Masse [g/mol] |
|---|---|---|
| Bz-FAARAG | 695,34 | 696,3 (+H⁺) |
| Bz-FRIVDARLEQVKAAGAY | 2010,07 | 2025,09 (+Na⁺) |
| Bz-FYtIVDAVLEQVKAAGAY | 1953,04 | 1954,31 (+H⁺) |
| Bz-FKVVFSAPV LEPTGPLHTQ FGYHHKVLY RN | 3673,98 | 3690,50 (+Na⁺) |

| | | |
|---|---|---|
| * Angabe der monoisotopiscnen Massen | | |

### Beispiel 4 - Trypsin-katalysierte N-terminale Einführung der Markergruppe Biotin in Polypeptiden mit enzym spezifischen Spaltstellen ausgehend von Biotinyl-OGp und Polypeptiden verschiedener Länge und Sequenz in der ionischen Flüssigkeit 4-Methyl N butyl-pyridinium tetrafluorborat.

Die individuellen enzym-spezifischen Aminosäurereste sind jeweils durch Fettdruck hervorgehoben (Bz, Benzoyl; OGp, 4-Guanidinophenylester).

Die Reaktionsbedingungen entsprechen denjenigen von Beispiel 3. Geändert wurden lediglich die Konzentrationen von Carboxykomponente (Biotinyl-OGp: 4 mM) und Aminokomponente (jeweiliges Peptid 2 mM). Die mittels MALDI-ToF nach ihrer Isolierung aus der Reaktionslösung identifizierten Polypeptidprodukte sind mit den jeweils berechneten bzw. gefundenen Molekülmassen in Tabelle 4 aufgeführt. Die Selektivität der enzymatischen Biotinylierungsreaktionen wurde durch Verwendung von N-terminal acetylierten Peptidanaloga untersucht Das Ausbleiben einer Produktbildung in diesen Fällen wurde als Bestätigung einer ausschließlichen N-tertninalen Biotinyliemag gewertet.

**Tabelle 4:**

| Syntheseprodukt | berechnete Masse [g/mol]* | Gefundene Masse [g/mol] |
|---|---|---|
| Biotinyl-RIVDARLEQVKAAGAY | 1985,05 | 1986,31 (+H⁺) |
| Biotinyl-KWFSAPV LEPTGPLHTQ FGYHITKVLY RN | 3648,96 | 3649,67 (+H⁺) |

| | | |
|---|---|---|
| * Angabe der monoisotopischen Massen | | |

Das erhaltene MALDI-ToF Massenspektrum des Syntheseproduktes Biotinyl-RIVDARLEQVKAAGAY ist beispielhaft in Fig. 1 dargestellt. Die gefundene Molmasse von 1986,31 entspricht dem (M+⁺) Signal der mit 1985,05 berechneten monoisotopischen Molmasse des Peptidproduktes.

### Beispiel 5 - Chymotrypsin-katalysierte selektive Einführung der Markergruppe Biotin in Lysozym aus Hühnereiweis in der ionischen Flüssigkeit 4-Methyl-N-butyl-pyridinium tetrafluorborat.

Chymotrypsin besitzt eine relativ breite Substratspezifität, wobei das Enzym jedoch nach aromatischen Aminosäureresten bevorzugt spaltet. Die individuellen, im Lysozym vorkommenden aromatischen Aminosänrereste sind jeweils durch Fettdruck hervorgehoben (Hepes, *N*-[2-hydroxyethyl]piperazin-*N*'-[2-ethansulfonsäure]; OGp, 4-Guanidinophenylester).

Primärssequenz von Lysozym aus Hühnereiweis:
KVFGRCELAA AMKRHGLDNY RGYSLGNWVC QATNRNTDGS
TDYGILQINS RWWCNDGRTP GSRNLCNIPC SALLSSDITA
SVNCAKKIVS DGNGMNAWVA QAWIRGCRL

1 ml Reaktionslösung, die 4-Methyl-N butyl-pyridinium tetrafluorborat, 20% Hepes-Puffer (0,05 M, pH 8,0), 2 mM Biotinyl-OGp, 0,5 mM Lysozym und 10 µM Chymotrypsin enthält, wird bei 25 °C gerührt. Nach 120 min wird die Reaktionslösung mit 1 proz. Trifluoressigsäure in Methanol/Wasser (1:1, v/v) auf pH 2 gebracht Die mittels MALDI-ToF nach Produktisolierung gefundene Molmasse von 14589,06 des Syntheseproduktes entspricht dem (M+H⁺) Signal der theoretisch berechneten monoisotopischen Molmasse einfach biotinylierten Lysozyms.

Die in der vorstehenden Beschreibung, den Ansprüchen sowie den Zeichnungen offenbarten Merkmale der Erfindung können sowohl einzeln als auch in beliebigen Kombinationen für die Verwirklichung der Erfindung in verschiedenen Ausführungsformen eingesetzt werden.

## Patentansprüche

1. Verfahren zur Synthese von Peptiden, Peptidmimetika und/oder Proteinen und/oder zur selektiven N-terminalen Modifizierung von Peptiden, Peptidmimetika und/oder Proteinen, mit den Schritten:
a) Bereitstellen einer Aminokomponente, wobei die Aminokomponente mindestens eine Aminosäure aufweist,
b) Bereitstellen einer Carboxykomponente, wobei die Carboxykomponente eine enzymspezifische Abgangsgruppe an der Carboxylgruppe aufweist, und die Carboxykomponente eine Verbindung mit mindestens einer Aminosäure ist oder eine Verbindung mit mindestens einer Marker- oder Reportergruppe,
c) Umsetzen der Aminokomponente und der Carboxykomponente in einem Reaktionsmedium, welches ein oder mehrere ionische Flüssigkeiten aufweist, in Gegenwart einer Protease, Peptidase und/oder Hydrolase, wobei zwischen der Aminokomponente und der Carboxykomponente unter Abspaltung der Abgangsgruppe eine Peptidbindung gebildet wird.

2. Verfahren nach Anspruch 1, weiter umfassend als Schritt:
d) Anreichern und/oder Isolieren des erhaltenen Peptids, Peptidmimetikums und/oder Proteins nach an sich bekannten Verfahren.

3. Verfahren nach Anspruch 1 oder 2,
**dadurch gekennzeichnet,**
**dass** die Aminokomponente ein Polypeptid oder Protein ist.

4. Verfahren nach einem oder mehreren der vorhergehenden Ansprüche,
**dadurch gekennzeichnet,**
**dass** die Aminokomponente eine Größe von 1 bis 1000 Aminosäuren, bevorzugt 30 bis 500, noch bevorzugter 30 bis 250 Aminosäuren aufweist.

5. Verfahren nach einem oder mehreren der vorhergehenden Ansprüche,
**dadurch gekennzeichnet,**
**dass** das Carboxy-Ende der Aminokomponente geschützt oder ungeschützt in nicht aktivierter Form vorliegt.

6. Verfahren nach einem oder mehreren der vorhergehenden Ansprüche,
**dadurch gekennzeichnet,**
**dass** die N^{α}-Aminofunktion der Aminokomponente ungeschützt vorliegt, wobei diese *N*^{α}-Aminofunktion mit der Carboxyfunktion der Carboxykomponente reagiert.

7. Verfahren nach einem oder mehreren der vorhergehenden Ansprüche,
**dadurch gekennzeichnet,**
**dass** die Carboxykomponente ein Polypeptid oder Protein ist.

8. Verfahren nach einem oder mehreren der vorhergehenden Ansprüche,
**dadurch gekennzeichnet,**
**dass** die Carboxykomponente eine Größe von 1 bis 1000 Aminosäuren, bevorzugt 30 bis 500, noch bevorzugter 30 bis 250 Aminosäuren aufweist.

9. Verfahren nach einem oder mehreren der vorhergehenden Ansprüche,
**dadurch gekennzeichnet,**
**dass** die Carboxy-Gruppe der Carboxykomponte mit der Abgangsgruppe einen Carbonsäureester oder ein Carbonsäureamid bildet.

10. Verfahren nach einem oder mehreren der vorhergehenden Ansprüche,
**dadurch gekennzeichnet,**
**dass** die Abgangsgruppe der Carboxykomponente ausgewählt ist aus der Gruppe, umfassend unsubstituierte und substituierte -O-Alkyl-, -O-Aryl-, -S-Alkyl-, -S-Aryl-Reste, -NH-Alkyl-, -NH-Aryl-, -N,N-Dialkyl-, -N,N-Diaryl- und -N-Aryl-N-Alkyl-Reste.

11. Verfahren nach Anspruch 10,
**dadurch gekennzeichnet,**
**dass** die Abgangsgruppe der Carboxykomponente durch ein oder mehrere Carbonsäurereste, Sulfonsäurereste oder Sulfonate substituiert ist.

12. Verfahren nach einem oder mehreren der vorhergehenden Ansprüche,
**dadurch gekennzeichnet,**
**dass** die Abgangsgruppe ein 4-Guanidinophenyl-, 4-Amidinophenyl-, 4-Guanidinophenylthio-oder 4-Amidinophenylthio-Rest ist oder eine hierzu strukturhomologe Verbindung.

13. Verfahren nach einem oder mehreren der vorhergehenden Ansprüche,
**dadurch gekennzeichnet,**
**dass** die Abgangsgruppe an die Spezifität der verwendeten Protease, Peptidase und/oder Hydrolase angepasst wird.

14. Verfahren nach einem oder mehreren der vorhergehenden Ansprüche,
**dadurch gekennzeichnet, dass**
die die Abgangsgruppe enthaltende Carboxykomponente die folgende Struktur aufweist:
Y-(Xaa)ₙ-R
wobei Y = eine N-terminale Schutzgruppe oder H ist,
Xaa = eine beliebige α-Aminosäure, β-Aminosäure oder ein Derivat derselben oder eine Marker- oder Reportergruppe ist,
R eine Abgangsgruppe ist, insbesondere eine Abgangsgruppe ist, die ausgewählt ist aus der Gruppe umfassend unsubstituierte und substituierte -O-Alkyl-, -O-Aryl-, -S-Alkyl-, -S-Aryl-Reste, 4-Guanidmophenyl-, 4-Amidinophenyl-, 4-Guanidinophenylthio-, 4- / Amidinophenylthio-Reste, die jeweils durch Sulfonsäuregruppen oder Sulfonate substituiert sein können, sowie Strukturhomologe hiervon, n eine ganze zahe von 1 bis 1000, bevorzugt 30 bis 500, noch bevorzugter 30 bis 250 ist.

15. Verfahren nach einem oder mehreren der vorhergehenden Ansprüche,
**dadurch gekennzeichnet,**
**dass** die Carboxykomponente eine Marker- oder Reportergruppe ist, ausgewählt aus Carboxygruppen enthaltenden Fluoreszenzmarkern wie Fluorescein, Rhodamin, Tetramethylrhodamin, 2-Aminobenzoesäure; Carboxygruppen enthaltenden Isotopenlabeln wie ¹³C-, ¹⁵N- und ¹⁷O-enthaltenden Aminosäuren oder Peptidfragmenten; Carboxygruppen enthaltenden Spinlabeln, wie Nitroxidlabel enthaltende Aminosäure und Fettsäurederivate; Biotin;Carboxygruppen enthaltenden vernetzenden Mittel (cross-linking agents) wie Diazoacetat, Diazopyruvat, p-Nitrophenyl-3-diazopyruvat; 2-(1,2-Dithiolan-3-yl)acetat; N,N'-1,2-Phenylendimaleimid; N,N'-1,4-Phenylendimaleimid.

16. Verfahren nach einem oder mehreren der vorhergehenden Ansprüche,
**dadurch gekennzeichnet,**
**dass** die Schritte a) bis c) und wahlweise d) zwei- oder mehrfach durchgeführt werden, um sequenziell ein Polypeptid oder Protein, das eine Marker- oder Reportergruppe aufweisen kann, herzustellen.

17. Verfahren nach einem oder mehreren der vorhergehenden Ansprüche,
**dadurch gekennzeichnet,**
**dass** eine gemäß den Schritten a) bis c) und ggf. d) hergestellte Verbindung als Aminokomponente und eine andere gemäß den Schritten a) bis c) und ggf. d) hergestellte Verbindung als Carboxykomponente eingesetzt wird.

18. Verfahren nach einem oder mehreren der vorhergehenden Ansprüche,
**dadurch gekennzeichnet,**
**dass** das Reaktionsmedium ausschließlich ein oder mehrere ionische Flüssigkeiten aufweist.

19. Verfahren nach einem oder mehreren der Ansprüche 1 bis 17,
**dadurch gekennzeichnet,**
**dass** das Reaktionsmedium ein oder mehrere ionische Flüssigkeiten und weiterhin Wasser und/oder ein organisches Lösungsmittel und wahlweise übliche Zusätze umfasst.

20. Verfahren nach einem oder mehreren der vorhergehenden Ansprüche,
**dadurch gekennzeichnet,**
**dass** der Anteil der ionischen Flüssigkeiten im Reaktionsmedium 50 - 100 Vol.-%, bevorzugt 70 - 100 oder 80 **-** 100 Vol.-%, bevorzugter 90-100 Vol.-%, ebenfalls bevorzugt 95 bis 100 Vol.-% oder 95 - 99 Vol.-% beträgt.

21. Verfahren nach einem oder mehreren der vorhergehenden Ansprüche,
**dadurch gekennzeichnet, dass**
als Kationen der ionischen Flüssigkeiten quaternisierte Alkyl-Imidazolimn-lonen, quaternisierte Alkyl-Ammonium-Ionen, quaternisierte Alkyl-Pyridinium-lonen und/oder quaternisierte Alkyl-Phosphonium-Ionen eingesetzt werden.

22. Verfahren nach Anspruch 21,
**dadurch gekennzeichnet, dass**
die Alkylreste der ionischen Flüssigkeiten verzweigt oder unverzweigt sind und 1 - 20 C-Atome, bevorzugt 2 - 10 C-Atome, noch bevorzugter 4-6 C-Atome aufweisen.

23. Verfahren nach einem oder mehreren der vorhergehenden Ansprüche,
**dadurch gekennzeichnet, dass**
als ionische Flüssigkeiten 1-Ethyl-3-methylimidazolium-, 1-Butyl-3-methylimidazolium- und/oder 4-Methyl-N-butyl-pyridinium-Salze eingesetzt werden.

24. Verfahren nach einem oder mehreren der vorhergehenden Ansprüche,
**dadurch gekennzeichnet, dass**
als Anionen der ionischen Flüssigkeiten Chlorid, Bromid, Chloraluminat, Nitrat, Benzolsulfonat, Triflat (Trifluormethansulfonat), Tosylat und/oder Tetrafluorborat eingesetzt werden.

25. Verfahren nach einem oder mehreren der vorhergehenden Ansprüche,
**dadurch gekennzeichnet,**
**dass** als Protease eine Cysteinprotease oder Serinprotease verwendet wird.

26. Verwendung einer Protease, Peptidase und/oder Hydrolase zur Synthese und/oder N-terminalen Modifzierung von Peptiden, Peptidmimetika und Proteinen, wobei das Peptid, Peptidmimetikum und Protein oder deren N-terminal markierte Spezies durch Ligation einer Aminokomponente und einer Carboxykomponente hergestellt wird und die Carboxykomponente eine Abgangsgruppe aufweist.

## Claims

1. Process for the synthesis of peptides, peptide mimetics and/or proteins and/or for the selective N-terminal modification of peptides, peptide mimetics and/or proteins, comprising the steps:
a) preparation of an amino component, wherein the amino component comprises at least one amino acid,
b) preparation of a carboxy component, wherein the carboxy component comprises an enzyme-specific leaving group on the carboxyl group, and the carboxy component is a compound having at least one amino acid or a compound having at least one marker or reporter group,
c) reaction of the amino component and the carboxy component in a reaction medium comprising one or more ionic liquids, in the presence of a protease, peptidase and/or hydrolase, wherein a peptide bond is formed between the amino component and the carboxy component, with cleavage of the leaving group.

2. Process according to claim 1, further comprising the step:
d) concentration and/or isolation of the resulting peptide, peptide mimetic and/or protein by processes known *per se.*

3. Process according to claim 1 or 2,
**characterised in that**
the amino component is a polypeptide or protein.

4. Process according to one or more of the preceding claims,
**characterised in that**
the amino component has a size of from 1 to 1000 amino acids, preferably from 30 to 500 amino acids, more preferably from 30 to 250 amino acids.

5. Process according to one or more of the preceding claims,
**characterised in that**
the carboxy end of the amino component is protected or unprotected in unactivated form.

6. Process according to one or more of the preceding claims,
**characterised in that** the *N*^{α} amino function of the amino component is unprotected, this *N*^{α} amino function reacting with the carboxy function of the carboxy component.

7. Process according to one or more of the preceding claims,
**characterised in that**
the carboxy component is a polypeptide or protein.

8. Process according to one or more of the preceding claims,
**characterised in that**
the carboxy component has a size of from 1 to 1000 amino acids, preferably from 30 to 500 amino acids, more preferably from 30 to 250 amino acids.

9. Process according to one or more of the preceding claims,
**characterised in that**
the carboxy group of the carboxy component forms a carboxylic acid ester or a carboxylic acid amide with the leaving group.

10. Process according to one or more of the preceding claims,
**characterised in that**
the leaving group of the carboxy component is selected from the group comprising unsubstituted and substituted -O-alkyl, -O-aryl, -S-alkyl, -S-aryl radicals, -NH-alkyl, -NH-aryl, -N,N-dialkyl, -N,N-diaryl and -N-aryl-N-alkyl radicals.

11. Process according to claim 10,
**characterised in that**
the leaving group of the carboxy component is substituted by one or more carboxylic acid radicals, sulfonic acid radicals or sulfonates.

12. Process according to one or more of the preceding claims,
**characterised in that**
the leaving group is a 4-guanidinophenyl, 4-amidinophenyl, 4-guanidinophenylthio or 4-amidinophenylthio radical or a compound that is structurally homologous thereto.

13. Process according to one or more of the preceding claims,
**characterised in that**
the leaving group is adapted to the specificity of the protease, peptidase and/or hydrolase that is used.

14. Process according to one or more of the preceding claims,
**characterised in that**
the carboxy component containing the leaving group has the following structure:
Y-(Xaa)ₙ-R
wherein Y is an N-terminal protecting group or is H,
Xaa is any desired α-amino acid, β-amino acid or a derivative thereof or is a marker or reporter group,
R is a leaving group, in particular a leaving group selected from the group comprising unsubstituted and substituted -O-alkyl, -O-aryl, -S-alkyl, -S-aryl radicals, 4-guanidinophenyl, 4-amidinophenyl, 4-guanidinophenylthio, 4-amidinophenylthio radicals, each of which can be substituted by sulfonic acid groups or by sulfonates, as well as structural homologues thereof,
n is an integer of from 1 to 1000, preferably from 30 to 500, more preferably from 30 to 250.

15. Process according to one or more of the preceding claims,
**characterised in that**
the carboxy component is a marker or reporter group selected from carboxy-group-containing fluorescence markers such as fluorescein, rhodamine, tetramethylrhodamine, 2-aminobenzoic acid; carboxy-group-containing isotope labels such as ¹³C-, ¹⁵N- and ¹⁷O-containing amino acids or peptide fragments; carboxy-group-containing spin labels, such as nitroxide-label-containing amino acid and fatty acid derivatives; biotin; carboxy-group-containing cross-linking agents such as diazoacetate, diazopyruvate, p-nitrophenyl 3-diazopyruvate; 2-(1,2-dithiolan-3-yl)acetate; N,N'-1,2-phenylenedimaleimide; N,N'-1,4-phenylenedimaleimide.

16. Process according to one or more of the preceding claims,
**characterised in that**
steps a) to c) and optionally d) are carried out two or more times in order sequentially to prepare a polypeptide or protein which can comprise a marker or reporter group.

17. Process according to one or more of the preceding claims,
**characterised in that**
a compound prepared according to steps a) to c) and optionally d) is used as the amino component and a different compound prepared according to steps a) to c) and optionally d) is used as the carboxy component.

18. Process according to one or more of the preceding claims,
**characterised in that**
the reaction medium solely comprises one or more ionic liquids.

19. Process according to one or more of claims 1 to 17,
**characterised in that**
the reaction medium comprises one or more ionic liquids and also water and/or an organic solvent and optionally conventional additives.

20. Process according to one or more of the preceding claims,
**characterised in that**
the proportion of ionic liquids in the reaction medium is from 50 to 100 vol.%, preferably from 70 to 100 or from 80 to 100 vol.%, more preferably from 90 to 100 vol.%, likewise preferably from 95 to 100 vol.% or from 95 to 99 vol.%.

21. Process according to one or more of the preceding claims,
**characterised in that**
quaternised alkyl-imidazolium ions, quaternised alkyl-ammonium ions, quaternised alkyl-pyridinium ions and/or quaternised alkyl-phosphonium ions are used as cations of the ionic liquids.

22. Process according to claim 21,
**characterised in that**
the alkyl radicals of the ionic liquids are branched or unbranched and contain from 1 to 20 carbon atoms, preferably from 2 to 10 carbon atoms, more preferably from 4 to 6 carbon atoms.

23. Process according to one or more of the preceding claims,
**characterised in that**
1-ethyl-3-methylimidazolium, 1-butyl-3-methylimidazolium and/or 4-methyl-N-butyl-pyridinium salts are used as ionic liquids.

24. Process according to one or more of the preceding claims,
**characterised in that**
chloride, bromide, chloroaluminate, nitrate, benzenesulfonate, triflate (trifluoromethanesulfonate), tosylate and/or tetrafluoroborate are used as anions of the ionic liquids.

25. Process according to one or more of the preceding claims,
**characterised in that**
a cysteine protease or serine protease is used as the protease.

26. Use of a protease, peptidase and/or hydrolase in the synthesis and/or N-terminal modification of peptides, peptide mimetics and proteins, wherein the peptide, peptide mimetic and protein, or N-terminally labelled species thereof, is prepared by ligation of an amino component and a carboxy component, and the carboxy component comprises a leaving group.

## Revendications

1. Procédé pour la synthèse de peptides, de mimétiques peptidiques et/ou de protéines et/ou pour la modification sélective N-terminale de peptides, de mimétiques peptidiques et/ou de protéines, comprenant les étapes :
a) de préparation d'un composant amino, le composant amino présentant au moins un acide aminé ;
b) de préparation d'un composant carboxyle, le composant carboxyle présentant un groupe partant à spécificité enzymatique sur le groupe carboxyle ; et le composant carboxyle représentant un composé comprenant au moins un acide aminé ou un composé comprenant au moins un groupe faisant office de marqueur ou faisant office de rapporteur ;
c) la mise en réaction du composant amino et du composant carboxyle dans un milieu de réaction qui présente un ou plusieurs liquides ioniques, en présence d'une protéase, d'une peptidase et/ou d'une hydrolase, une liaison peptidique étant formée entre le composant amino et le composant carboxyle par la séparation du groupe partant.

2. Procédé selon la revendication 1, comprenant en outre comme étape:
d) l'enrichissement et/ou l'isolation du peptide obtenu, du mimétique peptidique obtenu et/ou de la protéine obtenue, conformément à des procédés connus en soi.

3. Procédé selon la revendication 1 ou 2, **caractérisé en ce que** le composant amino est un polypeptide ou une protéine.

4. Procédé selon une ou plusieurs des revendications précédentes, **caractérisé en ce que** le composant amino possède une dimension de 1 à 1000 acides aminés, de préférence de 30 à 500, de manière plus préférée de 30 à 250 acides aminés.

5. Procédé selon une ou plusieurs des revendications précédentes, **caractérisé en ce que** l'extrémité carboxyle du composant amino est présente à l'état protégé ou à l'état non protégé dans sa forme activée.

6. Procédé selon une ou plusieurs des revendications précédentes, **caractérisé en ce que** la fonction N^{α}-amino du composant amino est présente à l'état non protégé, cette fonction N^{α}-amino réagissant avec la fonction carboxyle du composant carboxyle.

7. Procédé selon une ou plusieurs des revendications précédentes, **caractérisé en ce que** le composant carboxyle est un polypeptide ou une protéine.

8. Procédé selon une ou plusieurs des revendications précédentes, **caractérisé en ce que** le composant carboxyle présente une dimension de 1 à 1000 acides aminés, de préférence de 30 à 500, de manière plus préférée de 30 à 250 acides aminés.

9. Procédé selon une ou plusieurs des revendications précédentes, **caractérisé en ce que** le groupe carboxyle du composant carboxyle forme avec le groupe partant un ester d'acide carboxylique ou un amide d'acide carboxylique.

10. Procédé selon une ou plusieurs des revendications précédentes, **caractérisé en ce que** le groupe partant du composant carboxyle est choisi parmi le groupe comprenant des radicaux -O-alkyle, -O-aryle, -S-alkyle, -S-aryle non substitués et substitués, des radicaux -NH-alkyle, -NH-aryle, -NN-dialkyle, -NN-diaryle et -N-aryl-N-alkyle.

11. Procédé selon la revendication 10, **caractérisé en ce que** le groupe partant du composant carboxyle est substitué par un ou plusieurs radicaux d'acides carboxyliques, un ou plusieurs radicaux d'acides sulfoniques ou un ou plusieurs sulfonates.

12. Procédé selon une ou plusieurs des revendications précédentes, **caractérisé en ce que** le groupe partant représente un radical 4-guanidinophényle, un radical 4-amidinophényle, un radical 4-guanidinophénylthio ou un radical 4-amidinophénylthio ou un composé de structure homologue à celle desdits radicaux.

13. Procédé selon une ou plusieurs des revendications précédentes, **caractérisé en ce que** le groupe partant est adapté à la spécificité de la protéase, de la peptidase et/ou de l'hydrolase utilisées.

14. Procédé selon une ou plusieurs des revendications précédentes, **caractérisé en ce que** le composant carboxyle contenant le groupe partant présente la structure suivante
Y-(Xaa)ₙ-R
où Y représente un groupe de protection de l'extrémité N-terminale ou un atome d'hydrogène
Xaa représente n'importe quel acide α-aminé, n'importe quel acide β-aminé dérivé ou encore un groupe faisant office de marqueur ou un groupe faisant office de rapporteur,
R représente un groupe partant, en particulier un groupe partant qui est choisi parmi le groupe comprenant des radicaux -O-alkyle, -O-aryle, -S-alkyle, -S-aryle, non substitués et substitués, un radical 4-guanidinophényle, un radical 4-amidinophényle, un radical 4-guanidinophénylthio, un radical 4-amidinophénylthio, qui peuvent être respectivement substitués par des groupes d'acides sulfoniques ou par des groupes sulfonates, ainsi que des homologues de structure desdits radicaux, n représentant un nombre entier de 1 à 1000, de préférence de 30 à 500, de manière plus préférée de 30 à 250.

15. Procédé selon une ou plusieurs des revendications précédentes, **caractérisé en ce que** le composant carboxyle est un groupe faisant office de marqueur ou faisant office de rapporteur, choisi parmi des groupes carboxyle contenant des marqueurs de fluorescence tels que la fluorescéine, la rhodamine, la tétraméthylrhodamine, l'acide 2-aminobenzoïque ; des groupes carboxyle contenant des marqueurs isotopiques tels que des acides aminés ou des fragments peptidiques contenant ¹³C, ¹⁵N et ¹⁷O -, des groupes carboxyle contenant des marqueurs de spins tels que des acides aminés et des dérivés d'acides gras contenant des marqueurs de type nitroxyde ; de la biotine ; des groupes carboxyle contenant des agents de réticulation (cross-linking agents) tels que le diazoacétate, le diazopyruvate, le p-nitrophényl-3-diazopyruvate ; le 2-(1,2-dithiolan-3-yl)acétate ; le N,N'-1,2-phénylènedimaléimide ; le N,N'-1,4-phénylènedimaléimide.

16. Procédé selon une ou plusieurs des revendications précédentes, **caractérisé en ce que** les étapes a) à c) et de manière facultative d) sont mises en oeuvre à deux reprises ou plus pour préparer de manière séquentielle un polypeptide ou une protéine qui peut présenter un groupe faisant office de marqueur ou faisant office de rapporteur.

17. Procédé selon une ou plusieurs des revendications précédentes, **caractérisé en ce qu'**on met en oeuvre un composé préparé conformément aux étapes a) à c) et de manière facultative d) pour faire office de composant amino et un autre composé préparé conformément aux étapes a) à c) et de manière facultative d) pour faire office de composant carboxyle.

18. Procédé selon une ou plusieurs des revendications précédentes, **caractérisé en ce que** le milieu de réaction présente à titre exclusif un ou plusieurs liquides ioniques.

19. Procédé selon une ou plusieurs des revendications 1 à 17, **caractérisé en ce que** le milieu de réaction comprend un ou plusieurs liquides ioniques et en outre de l'eau et/ou un solvant organique et de manière facultative des additifs habituels.

20. Procédé selon une ou plusieurs des revendications précédentes, **caractérisé en ce que** la fraction des liquides ioniques dans le milieu de réaction s'élève de 50 à 100 % en volume, de préférence de 70 à 100 ou de 80 à 100 % en volume, de préférence de 90 à 100 % en volume, de manière également préférée de 95 à 100 % en volume ou de 95 à 99 % en volume.

21. Procédé selon une ou plusieurs des revendications précédentes, **caractérisé en ce qu'**on met en oeuvre, pour faire office de cations des liquides ioniques, des ions quaternisés d'alkyle-imidazolinium, des ions quaternisés d'alkyle-ammonium, des ions quaternisés d'alkyle-pyridinium et/ou des ions quaternisés d'alkyle-phosphonium.

22. Procédé selon la revendication 21, **caractérisé en ce que** des radicaux alkyle des liquides ioniques sont ramifiés ou non ramifiés et présentent de 1 à 20 atomes de carbone, de préférence de 2 à 10 atomes de carbone, de manière encore plus préférée de 4 à 6 atomes de carbone.

23. Procédé selon une ou plusieurs des revendications précédentes, **caractérisé en ce qu'**on met en oeuvre, pour faire office de liquides ioniques, des sels du 1-éthyl-3-méthylimidazolium, du 1-butyl-3-méthylimidazolium et/ou du 4-méthyl-N-butyl-pyridinium.

24. Procédé selon une ou plusieurs des revendications précédentes, **caractérisé en ce qu'**on met en oeuvre, pour faire office d'anions des liquides ioniques, un chlorure, un bromure, un chloroaluminate, un nitrate, un benzènesulfonate, un triflate (trifluorométhanesulfonate), un tosylate et/ou un tétrafluoroborate.

25. Procédé selon une ou plusieurs des revendications précédentes, **caractérisé en ce qu'**on utilise comme protéase une protéase à cystéine ou une protéase à sérine.

26. Utilisation d'une protéase, d'une peptidase et/ou d'une hydrolase pour la synthèse et/ou pour la modification N-terminale de peptides, de mimétiques peptidiques et de protéines, le peptide, le mimétique peptidique et la protéine ou leurs espèces marquées à l'extrémité terminale étant préparés par ligature d'un composant amino et d'un composant carboxyle, et le composant carboxyle présentant un groupe partant.
